# EUROPEAN PATENT SPECIFICATION

(11) **EP 2 088 963 B1**
(45) Date of publication and mention of the grant of the patent: **23.03.2011**
(21) Application number: 07839632.2
(22) Date of filing: 17.10.2007
(51) Int. Cl.: A61F 2/06

(54) **BIFURCATION STENT DESIGN WITH OVER EXPANSION CAPABILITY**
GEGABELTES STENTDESIGN MIT ÜBEREXPANSIONSFÄHIGKEIT
STENT PRÉSENTANT UNE BIFURCATION À CAPACITÉ DE SUREXPANSION

(30) Priority: 16.11.2006 US 859420 P; 16.10.2007 US 873058
(43) Date of publication of application: 19.08.2009
(73) Proprietor: Boston Scientific Limited, Christ Church (BB)
(72) Inventor: MEYER, Michael P., Richfield, MN 55423 (US); BROOME, Thomas E., Prior Lake, MN 55372 (US); YADIN, Amnon, Kfar Vitkin (IL); GREGORICH, Daniel, St. Louis Park, MN 55416 (US); GROTHEIM, Kevin, St. Paul, MN 55114 (US); HEGG, Jens, Minneapolis, MN 55408 (US)
(74) Representative: Hauck Patent- und Rechtsanwälte
(86) International application number: PCT/US2007/022152
(87) International publication number: WO 2008/063328

(56) References cited:
- WO-A-2007/070140
- US-A1- 2004 059 406
- US-A1- 2004 267 352
- US-A1- 2006 155 362
- US-B1- 6 325 826

## Description

### BACKGROUND OF THE INVENTION

### Field of the Invention

This invention relates to implantable medical devices and their manufacture.

### Description of the Related Art

A stent is a medical device introduced to a body lumen and is well known in the art. Typically, a stent is implanted in a blood vessel at the site of a stenosis or aneurysm endoluminally, i.e. by so-called "minimally invasive techniques" in which the stent in a radially reduced configuration, optionally restrained in a radially compressed configuration by a sheath and/or catheter, is delivered by a stent delivery system or "introducer" to the site where it is required. The introducer may enter the body from an access location outside the body, such as through the patient's skin, or by a "cut down" technique in which the entry blood vessel is exposed by minor surgical means.

Stents, grafts, stent-grafts, vena cava filters, expandable frameworks, and similar implantable medical devices, collectively referred to hereinafter as stents, are radially expandable endoprostheses which are typically intravascular implants capable of being implanted transluminally and enlarged radially after being introduced percutaneously. Stents may be implanted in a variety of body lumens or vessels such as within the vascular system, urinary tracts, bile ducts, fallopian tubes, coronary vessels, secondary vessels, etc. Stents may be used to reinforce body vessels and to prevent restenosis following angioplasty in the vascular system. They may be self-expanding, expanded by an internal radial force, such as when mounted on a balloon, or a combination of self-expanding and balloon expandable (hybrid expandable).

Stents may be created by methods including cutting or etching a design from a tubular stock, from a flat sheet which is cut or etched and which is subsequently rolled or from one or more interwoven wires or braids.

Within the vasculature, it is not uncommon for stenoses to form at a vessel bifurcation. A bifurcation is an area of the vasculature or other portion of the body where a first (or parent) vessel is bifurcated into two or more branch vessels. Where a stenotic lesion or lesions form at such a bifurcation, the lesion(s) can affect only one of the vessels (i.e., either of the branch vessels or the parent vessel), two of the vessels or all three vessels. Many prior art stents however are not wholly satisfactory for use where the site of desired application of the stent is juxtaposed or extends across a bifurcation in an artery or vein such, for example, as the bifurcation in the mammalian aortic artery into the common iliac arteries.

Stents made for use in bifurcated regions are generally known. When treating a bifurcated vessel, it may be desirable to use a stent having a side branch opening configured to provide fluid communication between the primary vessel and a secondary or branch vessel of the bifurcation. A secondary or branch stent may be received within and/or be positioned adjacent to the side branch opening of the primary stent.

In some locations, a branch vessel may be oriented at an acute angle with respect to a primary vessel. When a stent includes portions which extend into both primary vessels and branch vessels, select elements of the stent may be subject to relatively large amounts of bending, stress and strain.

There remains a need for stents designed for use in bifurcated vessels.

US 2004/0267352 Al discloses a stent having a protruding branch portion for bifurcated vessels. Fig. 22 shows a stent 89 having a main stent body 14 and a branch portion 30. The stent 89 has a discontinuous support member 104 surrounding two concentric rings 86 and 88. The support member 104 has a generally elliptical shape and includes a plurality of discontinuities 106 along the perimeter.

WO 2007/070140 Al is comprised in the prior art according to Article 54(3) and (4) EPC 1973. WO 2007/070140 Al discloses a bifurcated stent that uses telescopic rings to support or form a side branch lumen.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### BRIEF SUMMARY OF THE INVENTION

The invention is directed to a bifurcation stent having a main branch portion and a side branch portion which interface about a frame. The stent has an unexpanded state in which the stent comprises a generally tubular wall defining a first lumen and an expanded state in which the said first lumen is radially enlarged and a side branch projects obliquely outward from the first lumen. The side branch defines a second lumen in fluid communication with the first lumen through an opening defined by the frame. The frame is expandable from a first configuration enclosing a first area to a second configuration enclosing a second area wherein the second area is larger than the first area.

In some embodiments, the frame has multiple undulations, for instance three or more undulations distributed around the circumference, the undulations each having an amplitude. The frame area can be expanded by reducing the amplitude of the undulations to provide additional area within the frame when expanded.

The frame area is stable within a first expansion pressure range, but expands to a second configuration when expansion pressure is exceeded.

In some embodiments in the first configuration, the frame encloses a primary area about which at least one curvilinear expansion element of the frame at least partially extends.

In some embodiments the frame utilizes expansion elements that when not needed for expansion, can be utilized as support struts for the side branch.

In some embodiments the frame incorporates portions of expansion columns of the contralateral main channel lumen to provide additional over expansion capabilities. In some cases the expansion column portions incorporated into the frame are further modified to provide additional expansion capability to the frame and/or additional support in the contralateral channel.

In some embodiments the frame incorporates closed shape cellular geometries to provide frame expansion capability.

In some embodiments the frame includes one or more locking mechanisms that permit frame expansion but resist or prohibit recompression of the frame after expansion, in some cases the force necessary to further expand the frame can be tailored by the design, number and location of such mechanisms incorporated into the frame.

According to the invention the frame includes at least one weaker member that connects a first adjacent end of the frame to a second adjacent end of the frame. In the second configuration, the weaker member allows the first adjacent end and the second adjacent end to be disposed further from one another than in the first configuration.

These and other embodiments which characterize the invention are pointed out with particularity in the claims annexed hereto and forming a part hereof. However, for further understanding of the invention, its advantages and objectives obtained by its use, reference should be made to the drawings which form a further part hereof and the accompanying descriptive matter, in which there is illustrated and described embodiments of the invention.

### BRIEF DESCRIPTION OF THE SEVERAL VIEWS OF THE DRAWING(S)

A detailed description of the invention is hereafter described with specific reference being made to the drawings.
Figures 1a and 1b are schematic representations of bifurcations stents having a fixed frame, illustrative of the prior art.
Figure 2a is a view of a stent frame in a first configuration according to an embodiment of the invention, in flat plan representation. Figure 2b is a view of the frame of Fig. 2a in an enlarged area configuration.
Figures 3a - 3t are views as in Fig 2a depicting alternate frames in first configurations according to the same embodiment of the invention.
Figure 4a is a view of a stent frame in a first configuration according to another embodiment of the invention, in flat plan representation.
Figure 4b is a view of the frame of Fig. 4a in an enlarged area configuration.
Figures 5a - 5n are views as in Fig 4a depicting alternate frames in first configurations according to the same embodiment of the invention.
Figure 6a is a view of a stent frame in a first configuration according to another embodiment of the invention, in flat plan representation. Figure 6b is a perspective view of the frame of Fig. 6a in an intermediate configuration.
Figure 6c is a perspective view of the frame of Figures 6a and 6b in an enlarged area configuration.
Figures 7a - 7b are views as in Fig 6a depicting alternate frames in first configurations according to the same example as Figs 6a-6c.
Figures 8a -8c are views, in flat plan representation, of alternate stent frames in a first configuration, according to an example which combines features of previous examples.
Figure 9a is a view, in flat plan representation, of a stent frame in a first configuration according to another example.
Figure 9b is a enlarged fragmentary view of portion 9b of Figure 9a, when the frame is in an enlarged area configuration.
Figures 9c - 9i are views as in Figure 9a depicting alternate frames according to the same example.
Figure 9j is a fragmentary view of an expansion element of a further alternate frame of the example of Figure 9a, when the frame is in first configuration.
Figure 9k is a view of the expansion element of Figure 9j when the frame is in an enlarged area configuration.
Figure 10a is a schematic flat plan view of a stent frame in a first configuration according to another example of the invention.
Figure 10b is a enlarged view of portion 10b of Figure 10a.
Figure 11a is a schematic flat plan view of a stent frame in a first configuration according to the invention.
Figures 11b - 11e are enlarged fragmentary views of alternate configurations for portion 11b-d of Figure 11a.
Figure 11e is a schematic view of a frame of the embodiment of Figure 11a interfaced with components of the main channel support structure of a bifurcated stent.
Figures 12a - 12p are flat plan views of segments of stents showing the frame interface with the main channel support structure, with details of the side channel support structure removed.
Figure 13a is a view of a perspective view of a stent with a frame in Fig. 12d and both main channel and side channel portions expanded, but with the frame in unenlarged first configuration.
Figure 13b shows is a perspective view of a portion of a stent of the invention with a frame as in Fig. 12c and both main and side channel portions expanded, and with the frame in an enlarged configuration.

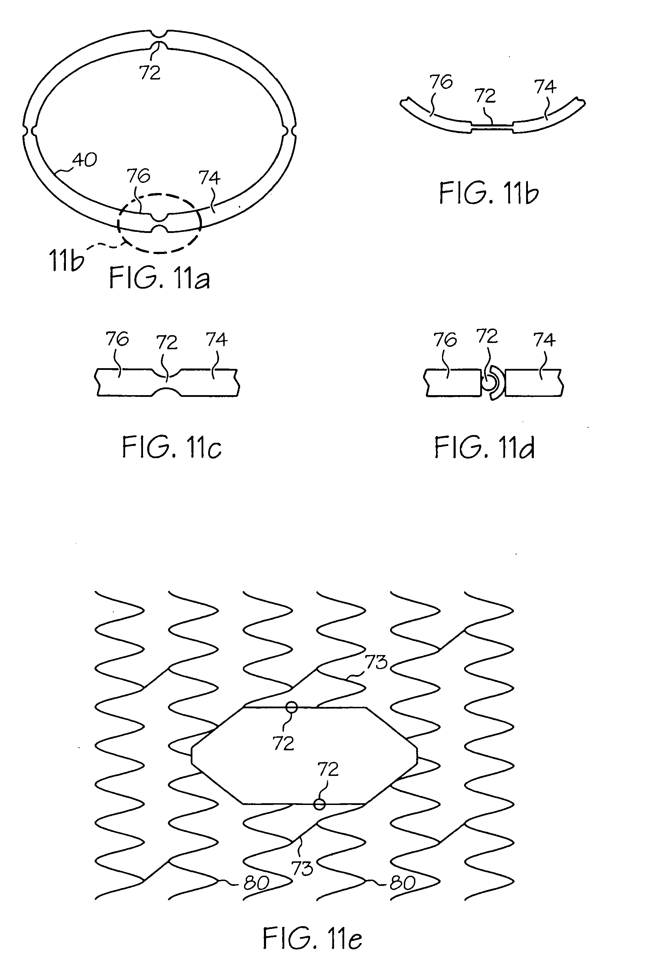

Figures 11a to 11e show an embodiment of the invention.

### DETAILED DESCRIPTION OF THE INVENTION

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

Except for the paragraph immediately below the use of the terms "unexpanded" and "expanded" in the Detailed Description are used in regard to the frame being expanded or unexpanded rather than the stent. It should be noted that as used below the term "first configuration" is substantially interchangeable with the frame being "unexpanded" and the term "second configuration" is substantially interchangeable with the frame being "expanded." The disclosure is focusing on inventive frames of the bifurcation stent that address use of the stent on a wide range of side vessel diameters and take off angles. The inventive frame geometries presented here are intended to be incorporated into any bifurcation stent regardless of unique side branch and main branch geometries. A side branch portion can extend from all the frames as shown and can include any number of geometries and constructs (e.g. serpentine portions, petal portions, finger portions). Some such structure of side branch portions is shown in US 2004-0088007 A1.

In some cases the main branch portion as described below is expanded before the frame is expanded as needed. In other cases the main branch portion and the frame expand with one another even as the side branch extends outward from the main branch portion.

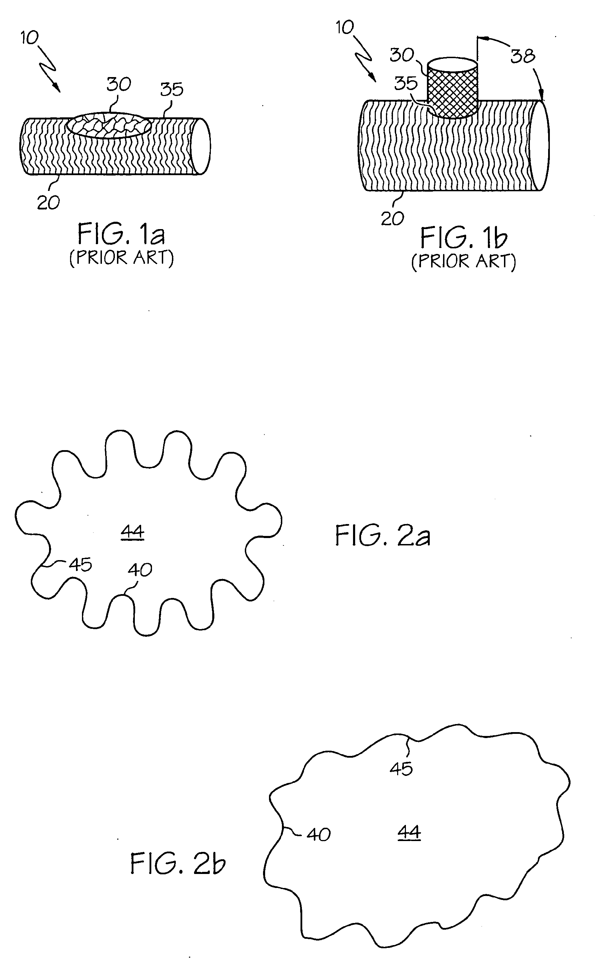

In Fig. 1a a schematic representation of bifurcation stent 10 in the unexpanded state is shown having a main branch portion 20 and an unexpanded side branch portion 30 with a frame 35 interconnecting the branches. In the expanded state of Fig. 1b the side branch portion 30 extends from the main branch portion 20. The side branch portion can extend at a take-off angle 38 that is an oblique angle from the main branch portion 20. For the purposes of this application, the term "oblique" refers to an angle of greater than zero degrees, such as an angle of between about I and about 180 degrees. An oblique angle explicitly includes angles of about 90 degrees and so includes acute, obtuse, and right angles.

Figs. 1a and 1b are illustrative of the prior art in that the frame 35 is shown as a simple closed circular or oval shape whose area is essentially unchanged as the stent is expanded. For any given bifurcation, the frame shape and size desirably would be such that the frame would be located just at the edge of the ostium of the side branch vessel. If the frame is located in this position the entire side branch geometry would he utilized to provide support to the side vessel. If the frame is too large some of the side branch geometry would be located outside the side branch opening, resulting in inefficient utilization of the very limited stent geometry with in the frame that is available for formation of the side branch. If the frame is too small for the ostium, part of the frame would hang into the ostium. Also, often the side branch vessel take off angle is more or less than 90 degrees from the main vessel. For non-right angle branches, the shape of the frame should be longer in the axial direction than in the circumferential direction to accommodate the elliptical ostium created when two cylinders meet at an angle. As the angle becomes more acute the major axis of the ellipse becomes larger resulting in the need for an even larger frame for the side branch geometry. A single frame configuration therefore is not ideally suited to these varied conditions.

In accordance with the present invention, bifurcation stent frame configurations are provided that allow for the frame area to be enlarged, and in at least some embodiments for its general shape to be substantially modified, to better accommodate the wide range of side branch ostia that are encountered at bifurcation locations. The frame area as described throughout this application refers to the area as shown in the plane or "flat" views of the frames and stents illustrated herein.

In some embodiments the enlarged area shape of the inventive frames may be obtained by a shape memory configuration. In other embodiments the frame is balloon enlargeable, which provides the advantage that the frame area may be better configured to the particular side branch ostium in which the stent is placed. Thus, with a balloon enlargeable frame, if a stent is delivered to a bifurcation having a side branch lumen cross-section that substantially matches the area enclosed by the frame and the take-off angle is not very acute then the frame need not be expanded or only somewhat expanded. However, in some instances if the take-off angle is very acute or if the side branch lumen cross-section is larger than the area enclosed by the initial frame shape, the frame may be easily expanded as needed to improve its fit to the side branch ostium.

According to some examples the frame has at least one undulated portion, the undulation(s) of which can be modified by reducing amplitude to thereby provide additional area within the frame when enlarged. Figures 2a and 2b illustrate these examples. The shape of the frame 40 in the unexpanded state of the stent is shown in Fig. 2a enclosing an area 44. Frame 40 has a multiplicity of undulations 45 around its circumference. The shape of the frame 40 may be modified to an enlarged configuration as illustrated in Fig. 2b wherein the amplitude of the undulations 45 is reduced, such that the area 44' enclosed by the frame 40 in the expanded configuration is greater than the area 44 enclosed by the frame 40 in the first configuration.

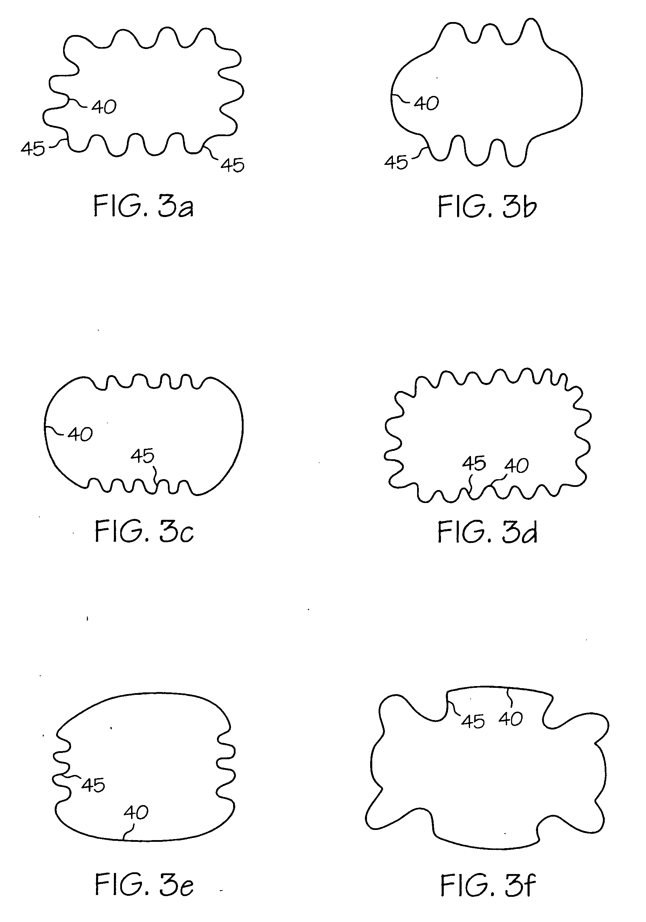

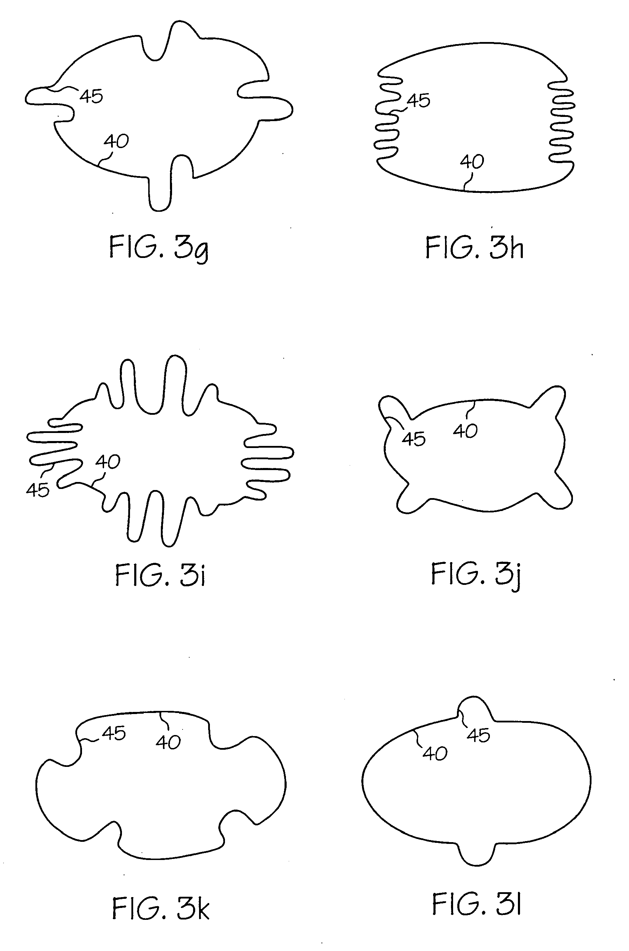

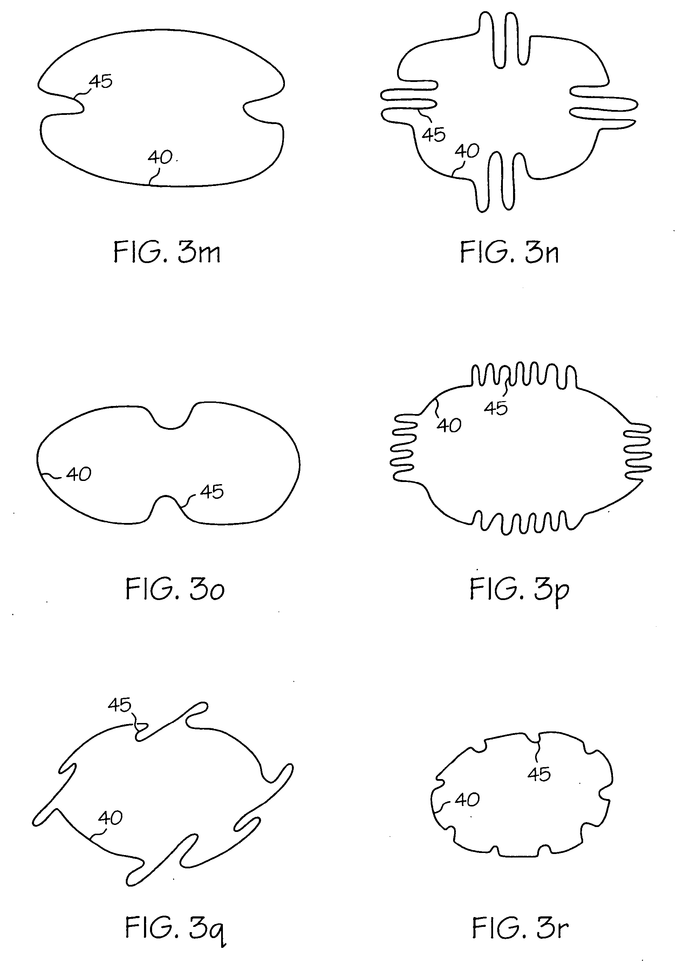

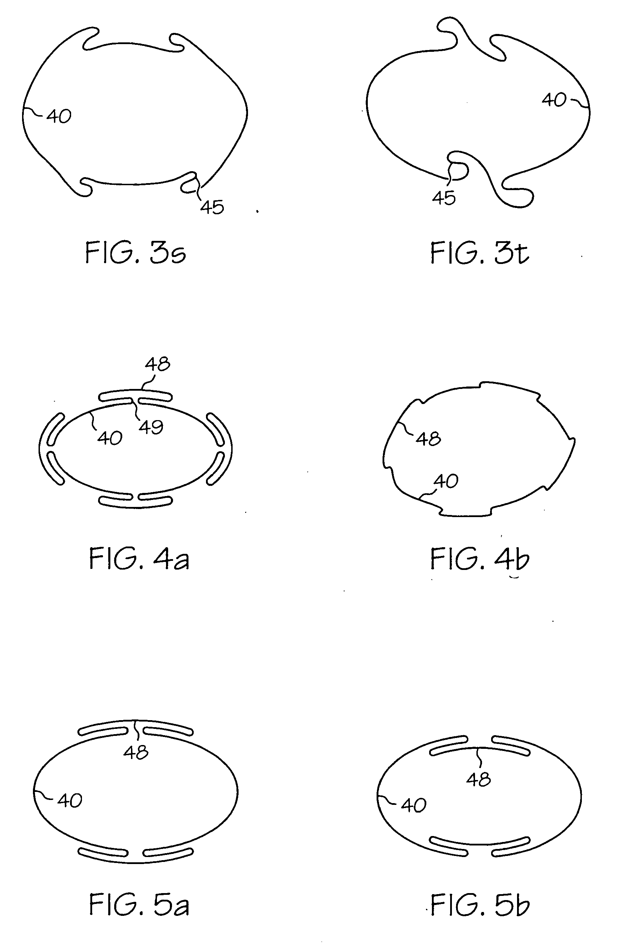

The initial configuration of an undulating frame can take a variety of configurations in which the undulations may be provided around the entire circumference of the frame or in only at particular locations. Figs. 3a - 3t depict alternate configurations for an unexpanded frame 40 in which to illustrate wide variety of undulations that may be implemented. Depending on location of the undulations in the frame, ratio of undulation amplitude to width, and thickness or width variation of the frame at different locations, the undulations can be made to enlarge area progressively with increasing enlargement on the frame, or resist area enlargement until a minimum enlargement pressure is exceeded.

Thus in some embodiments the frame is configured to provide a fixed or substantially fixed area up to a determined stress loading but then incrementally expand when stressed above the incremental load. This allows for a choreographed opening of the frame which is advantageous when the stent might be used for treating both small and large side branch diameters. Initial balloon expansion of the side branch will tend not to enlarge the frame, but then application of stress above an incremental minimum can be used to enlarge the frame in those cases where a large ostium is encountered.

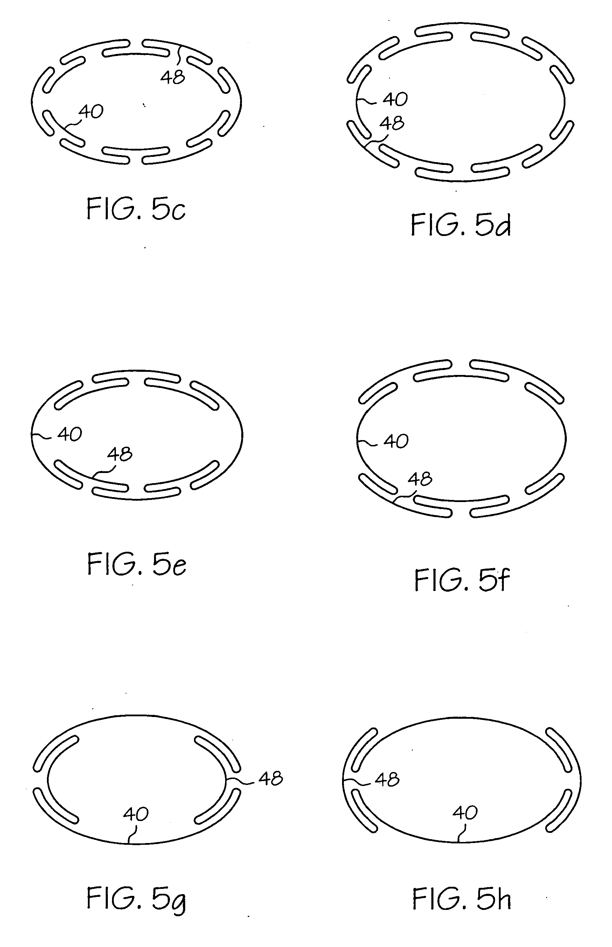

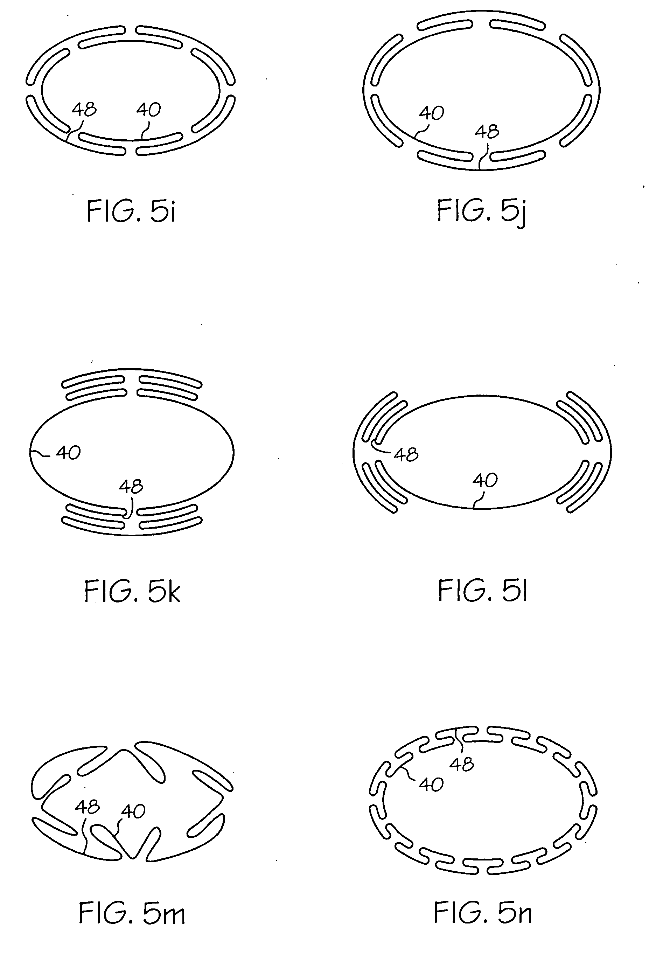

In some embodiments in the initial configuration, the frame encloses a primary area about which at least one elongate expansion element of the frame at least partially extends on the inside or outside of the primary area, or both. This is for example illustrated in Fig. 4a, where the enlargeable frame 40 substantially encloses an oval primary area but has with elongate expansion elements 48 connected to the primary area at narrow neck areas 49. The elongate expansion elements 48 of this frame may have a small width, and substantial length, which length may be substantially straight, or curvilinear. Suitably the elongate expansion elements 48 are substantially parallel to the curvature of the main body frame 40. Figure 4b shows an enlarged configuration of the frame 40 of Fig. 4a. The elongate expansion elements 48 can be located any number of locations and be either symmetric or asymmetric. Figs. 5a - 5n illustrate initial frame configurations of further embodiments of this type of frame.

The tight turns created from the small width of the elongate expansion elements 48 tend to hold their shape more strongly than if the expansion elements 48 had a larger turns of larger radius, because of this the frame will tend to maintain its initial shape upon initial deployment of the side branch elements. This allows for a choreographed opening of the frame as discussed above. In some cases a single balloon may have a sufficient range of size and strength to provide frame loads in the initial and incremental enlargement ranges. In others a second larger balloon may be needed to provide for the incremental enlargement.

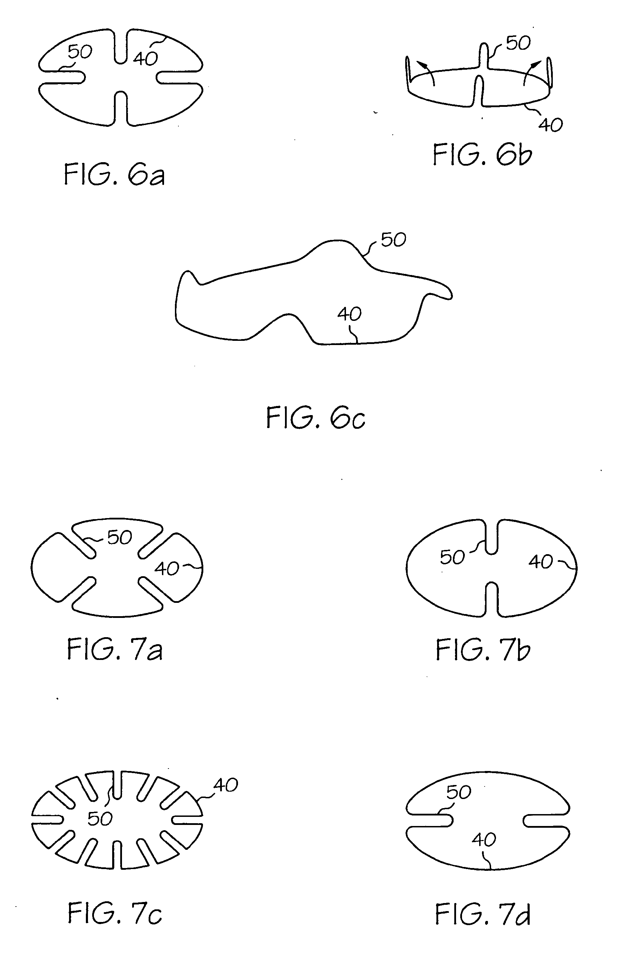

As shown in Figs. 6a - 6c, some examples of frame 40 utilize elongate expansion elements 50 that extend radially inward from the primary frame shape. If not used to enlarge the frame, elements 50 can be utilized as support struts for the side branch to provide vessel support and drug delivery. Fig. 6a depicts the frame 40 is in its initial configuration. Fig. 6b shows the expansion elements 50 rotated about the frame 40 and functioning as support struts. In cases where the stent frame may need to be enlarged, the expansion elements 50 provide additional frame length, which can be accessed by balloon expansion to provide an enlarged frame area. Fig. 6c illustrates such a configuration. The expansion elements 50 can be located at multiple locations around the frame 40. The frame can consist of one or more of these elements and the elements can vary in width and height depending on the application.

Figs. 7a- 7b illustrate additional frame geometries with expansion elements 50 having at least an elongate portion which is directed radially inward in the initial configuration that can be used that act as supports struts if frame enlargement is not required.

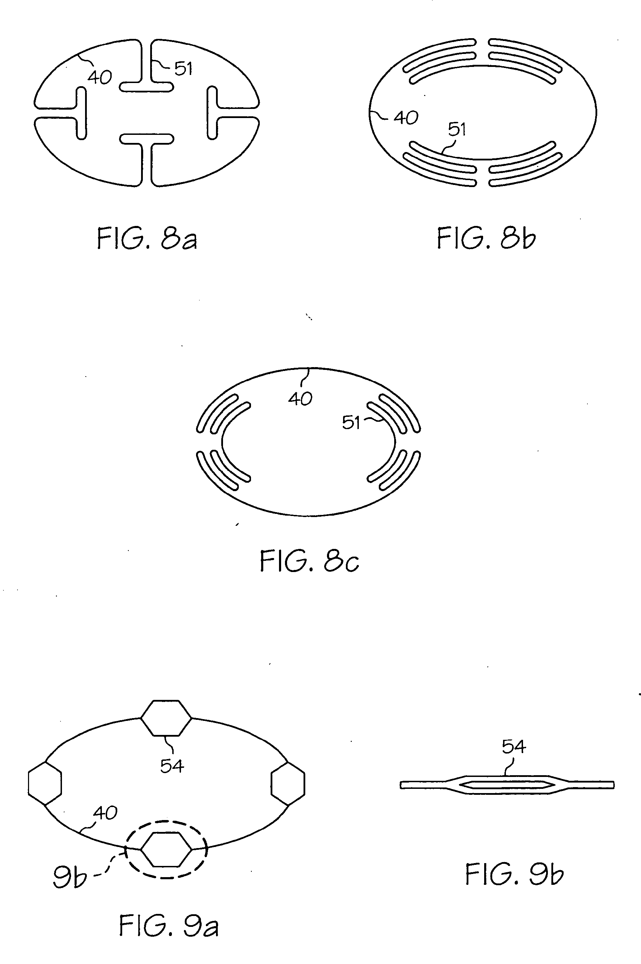

Frame geometries which employ expansion elements having features of both of the elongate elements 48 of Figs 4a and 5a-5n and of the inwardly directed elongate elements 50 of Figs. 6a and 7a - 7d may also be provided, for instance the elements 51 in Figs 8a - 8c.

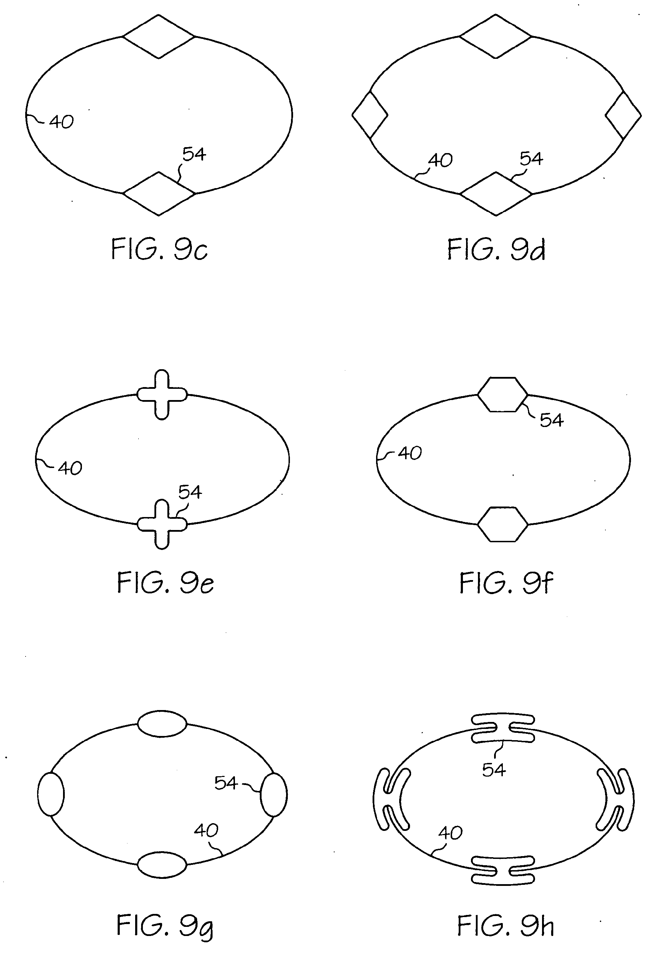

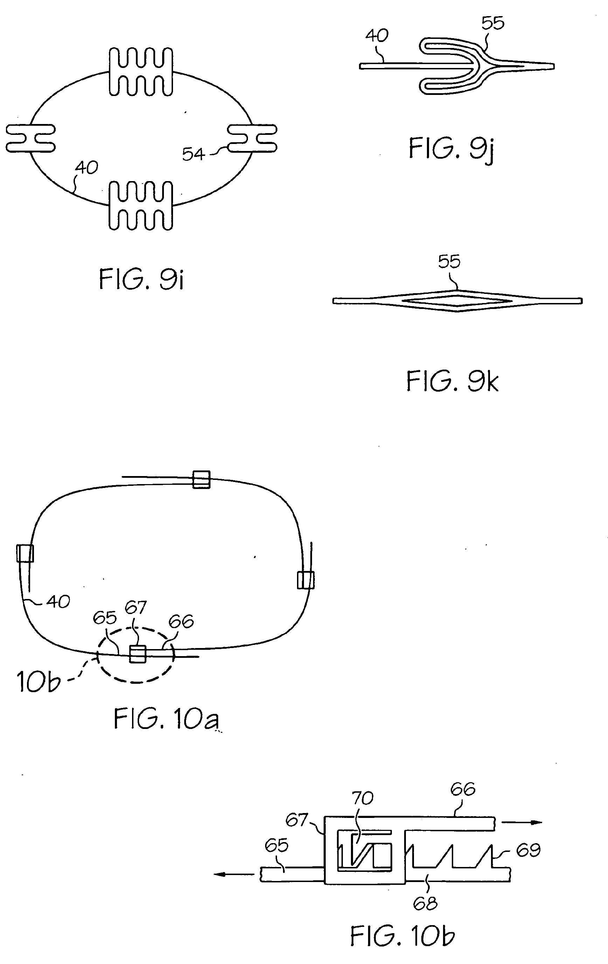

In some examples closed shape expansion elements may be used to provide additional perimeter which can be exploited to enlarge the frame area. In the initial frame configuration the frame splits, forms an expansion shape, and closes on the other side. If the frame needs further expansion the two ends of the expansion clement are allowed to pull apart while the sides are pulled in towards themselves. These structures also provide for a choreographed expansion of the frame in which the frame would tend to maintain its original shape under stress sufficient to initially deploy the side-branch but would yield under application of higher stresses if a further frame enlargement is needed. Figs. 9a and 9b illustrate one such construction. Fig 9a depicts a frame 40 with having hexagonal closed shape frame expansion element 54. Fig. 9b depicts the frame portion 9b of Fig 9a after the frame has been expanded to an enlarged configuration. Combinations of such features can also be employed. In further embodiments other closed geometry figures such as diamonds, circles, ovals, rectangles, and the like can be similarly employed. Figures 9c - 9i illustrate further frame configurations having closed geometry expansion elements incorporated therein.

Fig. 9j depicts a similar example of an expansion frame having a closed frame geometry in which the closed Y-shaped expansion elements 55 have an additional bends to add additional choreography to the opening of the side branch. The tight bends of the closed Y shape will maintain their shape upon initial deployment of the side branch but, if the frame needs additional expansion, a post-dilation balloon may be employed to deliver enough force to open the elements, as shown in Fig. 9k, resulting enlargement of the frame area.

In another example the frame has expansion elements that allow for frame enlargement by sliding an adjacent rail portion into the frame perimeter, the enlarged perimeter being maintained by a suitable sliding/locking structure such as the familiar "zip-tie" mechanism. Figs. 10a and 10b are illustrative of such embodiments. In Fig 10a frame 40 includes several such sliding/locking expansion elements. Fig 10b shows an enlarged view of one such sliding/locking expansion element. The frame perimeter is formed of rail portions 65 and 66 joined with a locking/sliding mechanism 67. The rail 66 includes a portion 68 which is collinear with rail 65, but outside the frame perimeter in the initial configuration of the frame. Upon application of enlargement pressure rail portions 65, 66 slide relative to each other in the direction of the arrows in Fig. 10b, incorporating some of rail portion 68 into the enlarged frame perimeter. The expanded size is maintained by the engagement of teeth 69 with the latch 70 in mechanism 67. Depending on the construction of the design of the sliding/locking mechanism 67 the force needed to further over expand the frame can be tailored depending on the application. Any number of sliding/locking geometries can be incorporated to allow the frame rails to slide colinear to each other. The frame can consist of one locking/sliding mechanism 67, or multiple mechanisms depending on the application.

As shown in Figs. 11a, a closed frame can be made with weak members that if needed, will break or yield inelastically when expansion stress is applied to the frame, allowing enlargement of the frame area: Upon general deployment the frame would maintain its designed shape. If the frame needed to expand beyond its designed shape the custom release features would break apart allowing the frame to be further expanded. In Fig. 11a at least one such weaker member 72 of the frame 40 connects one frame segment 74 to a second frame segment 76. In the expanded configuration breaking or elongating the weaker member 72 allows the frame segments 74, 76 to be disposed further from one another than in the first configuration. Enlarged details of several versions of such weak member portions are shown in Figs. 11b - 11d. In some embodiments, as shown in Figs 11b and 11c, the weaker member 72 has at least one portion that is thinner than frame segment 40a and frame segment 40b. Alternatively, the weaker member may have a different composition or have a different degree of hardening relative to the remainder of the frame segment. The weaker members 72 can be thin or notched struts designed to stretch or even break upon expansion of the frame 40. In Fig. 11d the weaker member 70 is a release junction that can include a portion that breaks when expansion pressure is applied to the frame but then opens expanded but stays in tact in an elongated state. As an alternative to enlargement by elongation of the weaker member, the weaker member may be adapted to break when the frame is subjected to expansion pressure, enlarging the frame by incorporating adjacent structure of the main stent body into the frame. Fig. 11e illustrates a frame 40 interfacing with expansion bands 80 of a stent main body portion. Weakened portions 72 are provided on the frame which are adapted to break when sufficiently pressurized, opening the frame to incorporate one or both of the stent portions 73 to become part of the frame.

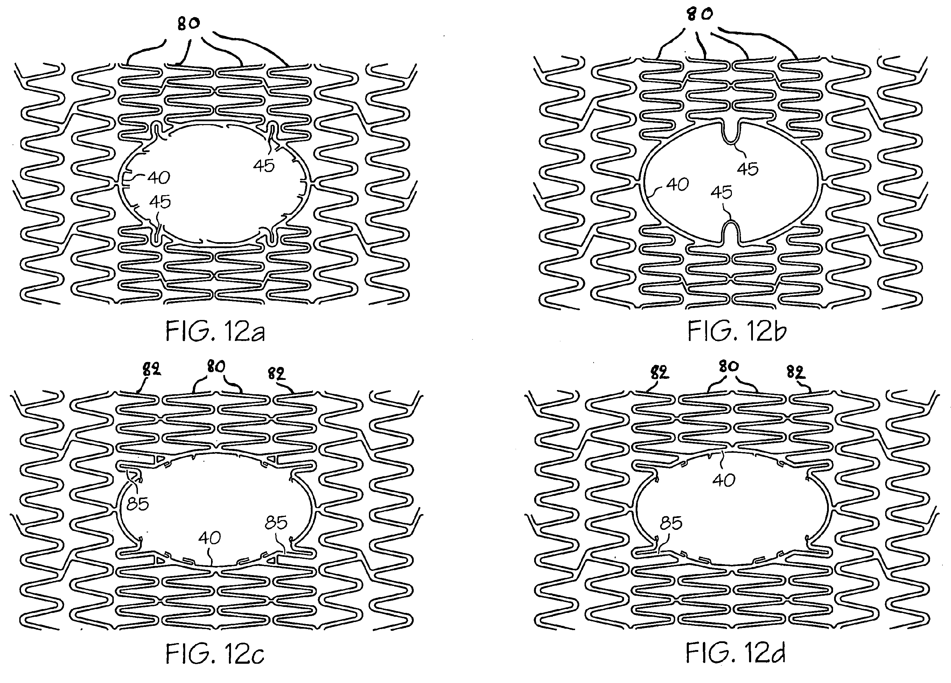

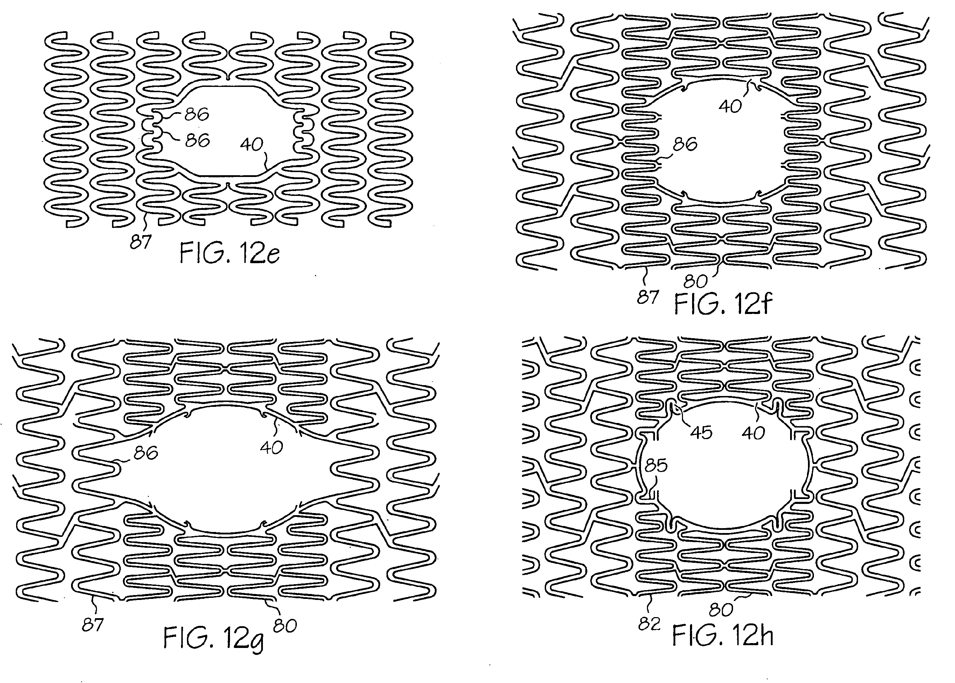

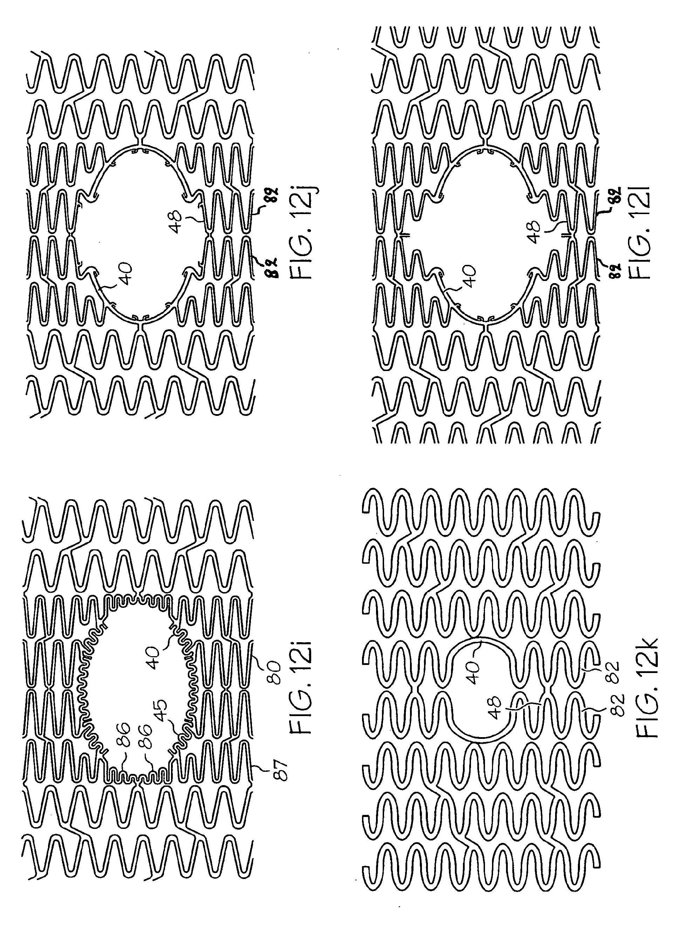

In providing an interface between the frame and the main body of the stent undulations or other expansion elements may be formed in a frame location distinct from the main body of the stent or they may be incorporated in a way that shares structure between the frame and structure of the main lumen portion of the stent such as circumferential bands. While the invention is not limited to any particular pattern or structure of the main and side lumen portions of the stent incorporating the inventive frames, Figures 12a - 12n are provided to illustrate a variety of interfaces which might be provided.

In Figures 12a and 12b, bands 80 are designated "contralateral" bands because, when deployed they extend around the main body lumen to provide vessel support opposite the frame. Contralateral bands 80, are distinctly terminated at the attachment to the frame 40, and the frame is provided with undulations 45 which allow for frame expansion if needed.

In Figs. 12c -12d, the frame undulations 85 are located in line with particular contralateral bands 82 and may be considered to be shared part of the bands 82 whereas other contralateral bands 80 terminate at the frame. In Fig. 12h, undulations 45 are distinct from the main channel bands attached to the frame, but the undulations 85 may also be considered to be part of the expansion band 82.

Frame patterns that incorporate a portion of a complete main lumen circumferential expansion column structure at proximal and distal ends of the frame are shown Figs. 12e -12g and 12i, where the undulations 86 are shared part of the expansion column 87 and of the frame 40. In Figures 12e and 12i, the frequency and wave length of the shared portion of the expansion column is changed in the portion of the column shared by the frame. Also in Fig 12i, the frame has additional undulations 45 not shared with an expansion column 87.

Between the proximal and distal ends of the frame, it is also possible to incorporate portions of main lumen expansion columns that form elongate expansion elements, as previously described, into a frame configuration to provide for flexibility in sizing and orienting the frame area. Figures 12j -12l are illustrative. In these figures adjacent contralateral expansion columns 82 form frame expansion elements 48 for the frame 40. Upon expansion of the frame 40 the expansion elements 48 can spread apart and provide additional cross-sectional area. When expansion of the frame is not necessary the expansion element 48 provides support within the main stent body as part of the contralateral bands 82.

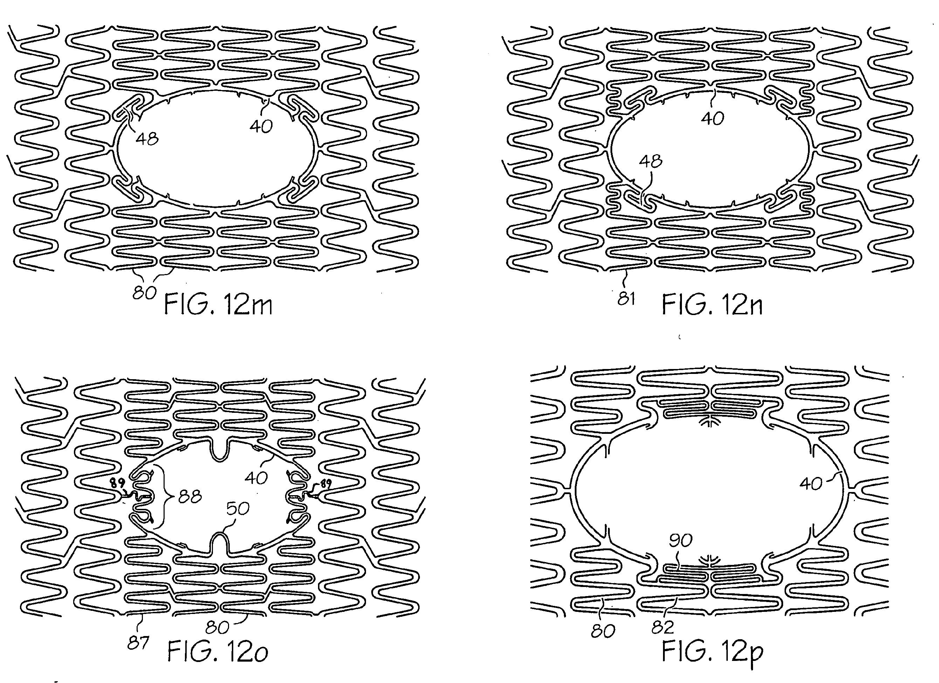

Figures 12m and 12n illustrate a simple frame interface with a main lumen structure in which the frame 40 includes elongate expansion elements 48 as previously described which are not shared parts of contralateral bands 80 or 81. In Fig, 12n a space accomodation for the expansion elements 48 has been made in expansion column 81 by changing the frequency and amplitude of its undulations near the frame attachment.

In Fig. 12o, the frame 40 includes an undulating region 88 that might be considered to be part of the expansion column 87, but is thinner so can be rotated out of plain to project partly into the side channel in the manner of the frames of Figs. 6a - 6c and 7a-7d. Bends in the thin side connector 89 also facilitate projection of this portion of the frame into side channel if the portion 88 is not needed for frame expansion. The undulations 50 can also be rotated into the side channel in the same manner.

Undulating portion 90 in Fig 12p is thinner relative to the frame 40 and main channel expansion bands 80, 82, providing for frame enlargement and/or rotation into the side channel as needed.

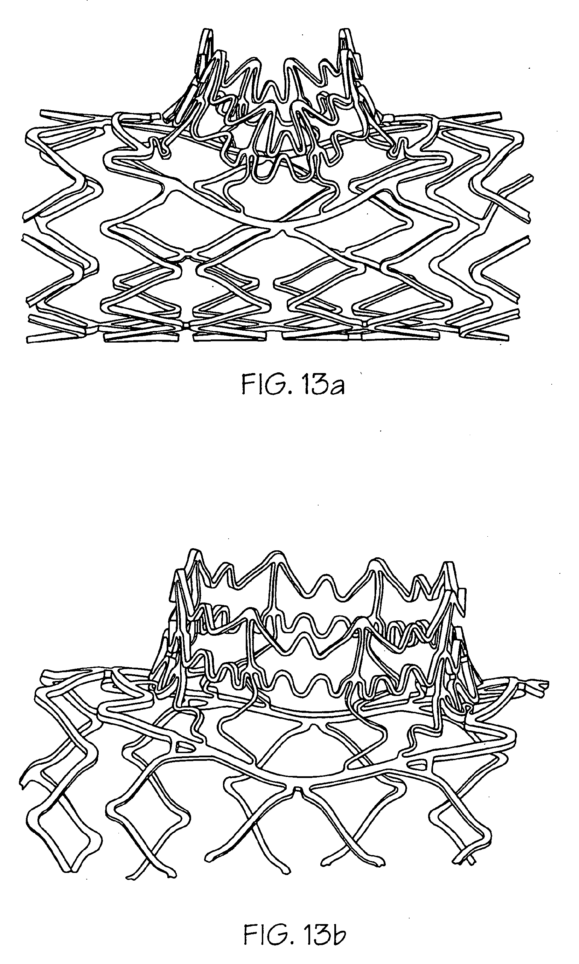

Figures 13a and 13b are perspective views of portions of bifurcated stents which employ frames of the invention corresponding to Figs. 12d and 12c, respectively. In the stent of Fig 13a the side channel projection has been formed, but without frame expansion. In the stent of Fig 13b the side channel has been expanded to a larger diameter with corresponding frame enlargement.

The inventive stents may be made from any suitable biocompatible materials including one or more polymers, one or more metals or combinations of polymer(s) and metal(s). Examples of suitable materials include biodegradable materials that are also biocompatible. By biodegradable is meant that a material will undergo breakdown or decomposition into harmless compounds as part of a normal biological process. Suitable biodegradable materials include polylactic acid, polyglycolic acid (PGA), collagen or other connective proteins or natural materials, polycaprolactone, hylauric acid, adhesive proteins, co-polymers of these materials as well as composites and combinations thereof and combinations of other biodegradable polymers. Other polymers that may be used include polyester and polycarbonate copolymers. Examples of suitable metals include, but arc not limited to, stainless steel, titanium, tantalum, platinum, tungsten, gold and alloys of any of the above-mentioned metals. Examples of suitable alloys include platinum-iridium alloys, cobalt-chromium alloys including Elgiloy and Phynox, MP35N alloy and nickel-titanium alloys, for example, Nitinol.

The inventive stents may be made of shape memory materials such as superelastic Nitinol or spring steel, or may be made of materials which are plastically deformable. In the case of shape memory materials, the stent may be provided with a memorized shape and then deformed to a reduced diameter shape. The stent may restore itself to its memorized shape upon being heated to a transition temperature and having any restraints removed therefrom.

The inventive stents may be created by methods including cutting or etching a design from a tubular stock, from a flat sheet which is cut or etched and which is subsequently rolled or from one or more interwoven wires or braids. Any other suitable technique which is known in the art or which is subsequently developed may also be used to manufacture the inventive stents disclosed herein.

In some embodiments the stent, the delivery system or other portion of the assembly may include one or more areas, bands, coatings, members, etc. that is (are) detectable by imaging modalities such as X-Ray, MRI, ultrasound, etc. In some embodiments at least a portion of the stent and/or adjacent assembly is at least partially radiopaque.

In some embodiments, at least a portion of the stent is configured to include one or more mechanisms for the delivery of a therapeutic agent. Often the agent will be in the form of a coating or other layer (or layers) of material placed on a surface region of the stent, which is adapted to be released at the site of the stent's implantation or areas adjacent thereto.

A therapeutic agent may be a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof Where the therapeutic agent includes a polymer agent, the polymer agent may be a polystyrene-polyisobutylene-polystyrene triblock copolymer (SIBS), polyethylene oxide, silicone rubber and/or any other suitable substrate.

This completes the description of the invention. Those skilled in the art may recognize other equivalents to the specific embodiment described herein which equivalents are intended to be encompassed by the claims attached hereto.

## Claims

1. A bifurcation stent (10) comprising a main branch portion (20) and a side branch portion (30), the main branch portion (20) and the side branch portion (30) interfacing about a frame (40), the stent (10) having an unexpanded state in which the stent (10) comprises a generally tubular wall defining a first lumen and an expanded state in which the said first lumen is radially enlarged and a side branch projects obliquely outward from the first lumen, the side branch defining a second lumen in fluid communication with the first lumen through an opening defined by said frame, wherein the frame (40) has a first stable configuration enclosing a first area (44) when subjected to frame expansion stress within a first pressure range and the frame (40) expands to a second configuration enclosing a second area larger than the first area when the frame is subjected to expansion stress above the first pressure range, **characterized in that** the frame (40) includes at least one weaker member (72) that connects a first adjacent end (74) of the frame (40) to a second adjacent end (76) of the frame (40), in the second configuration the weaker member (72) allows the first adjacent end (74) and the second adjacent end (76) to be disposed further from one another than in the first configuration.

2. The bifurcation stent of claim 1 wherein the frame in the first configuration substantially encloses a primary area which opens into one or more narrow elongated expansion elements via relative narrow neck areas.

3. The bifurcation stent of claim 1 wherein the frame includes at least one closed shape geometry portion that is collapsible to enlarge the frame area.

4. The bifurcation stent of claim 3 wherein the at least one closed shape geometry portion forms a polygon.

5. The bifurcation stent of claim 1 wherein the frame includes at least mechanism that allows for frame enlargement by sliding an adjacent rail portion of the stent into the frame perimeter upon application of expansion pressure to the frame.

6. The bifurcation stent of claim 1 wherein the frame has portions that are shared with an expansion column of the main body portion.

7. The bifurcation stent of claim 6 wherein a shared portion of the frame and a body expansion column has undulations with substantially same amplitude and wavelength as that of the expansion columns.

8. The bifurcation stent of claim 6 wherein a shared portion of the frame and a body expansion column has undulations with substantially different amplitude and/or wavelength as that of the expansion columns.

9. The bifurcation stent of claim 6 wherein an elongated expansion element of said frame is shared with a portion of a contralateral expansion column of said main branch portion.

## Patentansprüche

1. Abzweigungsstent (10) umfassend einen Hauptzweigabschnitt (20) und einen Seitenzweigabschnitt (30), wobei der Hauptzweigabschnitt (20) und der Seitenzweigabschnitt (30) über einen Rahmen (40) miteinander verbunden sind, der Stent (10) einen nicht expandierten Zustand aufweist, in welchem der Stent (10) eine ein erstes Lumen definierende allgemein röhrenförmige Wand umfasst, und einen expandierten Zustand, in welchem das erste Lumen radial vergrößert ist und ein Seitenzweig von dem ersten Lumen schräg nach außen vorsteht und der Seitenzweig ein zweites Lumen definiert, das über eine durch den Rahmen definierte Öffnung in Fluidkommunikation mit dem ersten Lumen ist, wobei der Rahmen (40) eine erste stabile Konfiguration aufweist, die eine erste Fläche umschließt, wenn er einer Rahmen-Expansionsspannung innerhalb eines ersten Druckbereichs unterworfen wird, und der Rahmen (40) sich zu einer zweiten Konfiguration ausdehnt, die eine zweite Fläche größer als die erste Fläche umschließt, wenn der Rahmen einer Rahmen-Expansionsspannung oberhalb des ersten Druckbereichs unterworfen wird, **dadurch gekennzeichnet dass** der Rahmen (40) mindestens ein schwächeres Glied (72) beinhaltet, welches ein erstes angrenzendes Ende (74) des Rahmens (40) mit einem zweiten angrenzenden Ende (76) des Rahmens (40) verbindet und dass in der zweiten Konfiguration das schwächere Glied (72) dem ersten angrenzenden Ende (74) und dem zweiten angrenzenden Ende (76) ermöglicht, weiter voneinander angeordnet zu sein als in der ersten Konfiguration.

2. Abzweigungsstent gemäß Anspruch 1, wobei der Rahmen in der ersten Konfiguration im wesentlichen eine Primärfläche umschließt, welche sich über verhältnismäßig enge Halsflächen in eines oder mehrere enge, längliche Expansionselemente öffnet.

3. Abzweigungsstent gemäß Anspruch 1, wobei der Rahmen mindestens einen Abschnitt mit geschlossenförmiger Geometrie beinhaltet, welcher zur Vergrößerung der Rahmenfläche zusammenfaltbar ist.

4. Abzweigungsstent gemäß Anspruch 3, wobei der mindestens eine Abschnitt mit geschlossenförmiger Geometrie ein Vieleck bildet.

5. Abzweigungsstent gemäß Anspruch 1, wobei der Rahmen mindestens einen Mechanismus beinhaltet, welcher durch Verschieben eines angrenzenden Schienenabschnitts des Stents in die äußere Rahmenbegrenzung mittels Aufbringen von Expansionsdruck auf den Rahmen eine Rahmenvergrößerung ermöglicht.

6. Abzweigungsstent gemäß Anspruch 1, wobei der Rahmen Abschnitte aufweist, welche er mit einer Expansionssäule des Hauptkörperabschnitts teilt.

7. Abzweigungsstent gemäß Anspruch 6, wobei ein gemeinsamer Abschnitt des Rahmens und einer Körperexpansionssäule Welligkeiten mit im wesentlichen gleicher Amplitude und Wellenlänge wie diejenige der Expansionssäulen aufweist.

8. Abzweigungsstent gemäß Anspruch 6, wobei ein gemeinsamer Abschnitt des Rahmens und einer Körperexpansionssäule Welligkeiten mit im wesentlichen verschiedener Amplitude und/oder Wellenlänge wie diejenige der Expansionssäulen aufweist.

9. Abzweigungsstent gemäß Anspruch 6, wobei ein längliches Expansionselement des Rahmens Teilhabe an einem Abschnitt einer Expansionssäule auf der entgegen gesetzten Seite des Hauptzweigabschnitts hat.

## Revendications

1. Stent bifurqué (10) comportant une partie de branche principale (20) et une partie de branche latérale (30), la partie de branche principale (20) et la partie de branche latérale (30) étant reliées l'une à l'autre à travers un cadre (40), le Stent (10) ayant une condition non dilatée, dans laquelle le Stent (10) comporte une paroi généralement tubulaire qui définit une première lumière, et une condition dilatée, dans laquelle la première lumière est radialement agrandie et une branche latérale obliquement saillit de la première lumière vers l'extérieur, la branche latérale définissant une deuxième lumière en communication fluidique avec la première lumière à travers une ouverture définie par le cadre, le cadre (40) ayant une première configuration stable entourant une première aire (44) quand il est soumis à une contrainte de dilatation de cadre dans une première gamme de pression, le cadre (40) se dilatant à une deuxième configuration qui entoure une deuxième aire plus grande que la première aire quand le cadre est soumis à une contrainte de dilatation de cadre au-dessus de la première gamme de pression, **caractérisé en ce que** le cadre (40) inclut au moins un membre plus faible (72), qui relie une première extrémité adjacente (74) du cadre (40) avec une deuxième extrémité adjacente (76) du cadre (40), et que dans la deuxième configuration le membre plus faible (72) permet à la première extrémité adjacente (74) et à la deuxième extrémité adjacente (76) d'être disposé plus loin l'un de l'autre que dans la première configuration.

2. Stent bifurqué selon la revendication 1, dans lequel le cadre essentiellement entoure une aire primaire dans la première configuration, qui s'ouvre dans un ou plusieurs éléments de dilatation étroits allongés via des aires de col relativement étroites.

3. Stent bifurqué selon la revendication 1, dans lequel le cadre inclut au moins une partie à géométrie en forme close, qui est pliable pour agrandir l'aire du cadre.

4. Stent bifurqué selon la revendication 3, dans lequel l'au moins une partie à géométrie en forme close forme un polygone.

5. Stent bifurqué selon la revendication 1, dans lequel le cadre inclut au moins un mécanisme qui permet un agrandissement du cadre en mouvant une partie de rail du Stent adjacente vers la périphérie du cadre par application de force de dilatation au cadre.

6. Stent bifurqué selon la revendication 1, dans lequel le cadre a des parties qui sont en commun avec une colonne de dilatation de la partie de corps principale.

7. Stent bifurqué selon la revendication 6, dans lequel une partie en commun avec le cadre et avec une colonne de dilatation du corps a des ondulations avec essentiellement la même amplitude et longueur d'onde que celles des colonnes de dilatation.

8. Stent bifurqué selon la revendication 6, dans lequel une partie en commun avec le cadre et avec une colonne de dilatation du corps a des ondulations avec une amplitude et/ou longueur d'onde essentiellement différente de celles des colonnes de dilatation.

9. Stent bifurqué selon la revendication 6, dans lequel un élément de dilatation allongé du cadre est en commun avec une partie d'une colonne de dilatation sur le côté opposé de ladite partie de branche principale.
